# EUROPÄISCHE PATENTSCHRIFT

(11) **EP 3 328 478 B1**
(45) Veröffentlichungstag und Bekanntmachung des Hinweises auf die Patenterteilung: **25.01.2023**
(21) Anmeldenummer: 16748085.4
(22) Anmeldetag: 26.07.2016
(51) Int. Cl.: A61M 25/06, A61B 17/34, A61B 90/40

(54) **PUNKTIONSSYSTEM**
PUNCTURING SYSTEM
SYSTÈME DE PONCTION

(30) Priorität: 29.07.2015 DE 102015112388; 21.10.2015 DE 102015117923
(43) Veröffentlichungstag der Anmeldung: 06.06.2018
(73) Patentinhaber: Ebnet Medical GmbH, 19055 Schwerin (DE); Mölgen, Roland, 30163 Hannover (DE); Raymondos, Konstantinos, 30655 Hannover (DE)
(72) Erfinder: Ebnet, Jens, 19055 Schwerin (DE); Mölgen, Roland, 30163 Hannover (DE); Raymondos, Konstantinos, 30655 Hannover (DE)
(74) Vertreter: Gramm, Lins & Partner Patent- und Rechtsanwälte PartGmbB
(86) Internationale Anmeldenummer: PCT/EP2016/067785
(87) Internationale Veröffentlichungsnummer: WO 2017/017095

(56) Entgegenhaltungen:
- WO-A1-99/37354
- WO-A1-2011/089014
- WO-A1-2012/162677
- WO-A1-2013/138519
- DE-A1-102008 045 692
- US-A1- 2004 147 877

## Beschreibung

Die Erfindung betrifft ein Punktionssystem mit einem äußeren tubusförmigen Körper, der zum Verweilen in einem Körperteil eines Lebewesens eingerichtet ist, wie z.B. einen zentralvenösen Katheter mit einem Katheterschlauch, der zum Verweilen in einer Vene eingerichtet ist.

Im Hinblick auf Jurisdiktionen, in denen Behandlungsverfahren dem Patentschutz zugänglich sind, betrifft die Erfindung ferner verbesserte Verfahren zum Applizieren eines Punktionssystems wie z.B. eines zentralvenösen Katheters, insbesondere unter Verwendung des erfindungsgemäßen Punktionssystems wie z.B. des zentralvenösen Katheters.

Der zentralvenöse Katheter wird auch als zentraler Venenkatheter oder Zentralvenenkatheter bzw. zentralvenöser Zugang bezeichnet, oder abgekürzt ZVK.

Die Durchführung einer zentralvenösen Katheterisierung ist eine der häufigsten ärztlichen Maßnahmen überhaupt. Ein ZVK ist ein Katheter, welcher über eine größere punktable nicht unmittelbar herznahe sogenannte "periphere Vene" eingeführt wird und dessen Spitze herznah in einer "zentralen Vene" zum Liegen kommt. Über ihn können sowohl Flüssigkeiten und Blutprodukte infundiert als auch Medikamente injiziert werden. Außerdem können Blutproben gewonnen und der Venendruck gemessen werden. Ein zentralvenöser Katheter kann bei komplikationslosem Verlauf wochenlang im Patienten verbleiben.

Die Anlage eines zentralvenösen Katheters stellt ein Routineverfahren in der innerklinischen Notfallmedizin, der Anästhesiologie und Intensivmedizin dar. Aber auch in anderen klinischen Bereichen findet er Verwendung, so z.B. in der Onkologie zur Applikation von Chemotherapeutika und in der Intensivmedizin zur hochkalorischen parenteralen Ernährung, z.B. nach Magen-Darm-Operationen.

Über einen zentralvenösen Katheter können kreislaufstabilisierende Medikamente und andere Substanzen herznah appliziert werden, wo sie unmittelbar wirksam sind. Stark konzentrierte Infusionslösungen wie Elektrolytlösungen und Ernährungslösungen können häufig nur über einen zentralvenöser Katheter verabreicht werden, da sie kleinere Venen (z.B. Handrücken- und Armvenen) zu stark reizen und somit zu Entzündungen und Thrombosen derselben führen würden.

Im Regelfall hat ein zentralvenöser Katheter patientenfern ein bis fünf sogenannte "Schenkel" (Lumen), die sich zum Patienten hin in einem gemeinsamen Katheterschlauch vereinigen, welcher in die Vene eingebracht wird. Er besteht aus körperverträglichem Material wie z.B. Polyurethan, welches ihm zudem thermoplastische Eigenschaften verleiht. Der Katheterschlauch wird also bei Erwärmung auf Körpertemperatur weicher. Die Einführtiefe des Katheterschlauchs ab Hautniveau beträgt bei erwachsenen Patienten ca. 15-20 cm, abhängig vom gewählten Zugangsweg und von patientenspezifischen Faktoren wie Größe und Gewicht.

Ein ZVK wird zumeist mittels der 1953 beschriebenen Seldinger-Technik mit einem Seldinger-Draht in den Körper eingebracht. Diese Technik hat einige Nachteile. Aufgrund der Notwendigkeit mehrerer sequentieller Arbeitsschritte und eines sterilen Arbeitsfeldes besteht eine Verletzungs- und Infektionsgefahr für Patient und Anwender. Der Seldinger-Draht kann das Herz schädigen und Herzrhythmusstörungen hervorrufen, zudem akzidentiell im Patienten verbleiben. Seine Lage im Körper kann unklar sein. Nicht zuletzt ist die Seldinger-Technik komplex in der Durchführung und mit hohen Kosten verbunden. Eine Anwendung dieses Verfahrens außerhalb der Klinik und bei sehr beengten Platzverhältnissen ist daher nahezu unmöglich. Bisher existierende Direktpunktionstechniken ohne Seldinger-Draht haben sich in der breiten klinischen Anwendung aufgrund des nur in begrenztem Durchmesser einführbaren Katheterschlauchs nicht durchsetzen können.

Aus der WO 2011/089014 A1 ist ein Katheter mit Abstandshalter bekannt. Aus der US 2004/0147877 A1 ist ein Kathetereinführungssystem bekannt. Aus der WO 2012/0162677 A1 ist eine Zugangseinrichtung bekannt. Aus der WO 2013/138519 A1 ist ein flexibler medizinischer Artikel und ein Verfahren zu dessen Herstellung bekannt. Aus der WO 99/37354 A1 ist ein Kanüleneinführungssystem bekannt. Aus der DE 10 2008 045 692 A1 ist eine Vorrichtung und ein Verfahren zum Anlegen eines Zugangs zu einem Hohlorgan sowie ein Werkzeug bekannt.

Der Erfindung liegt daher die Aufgabe zugrunde, ein Punktionssystem wie z.B. einen zentralvenösen Katheter anzugeben, das einfacher und schneller am Patienten appliziert werden kann.

Im Hinblick auf Jurisdiktionen, in denen Behandlungsverfahren dem Patentschutz zugänglich sind, liegt der Erfindung ferner die Aufgabe zugrunde, verbesserte Verfahren zum Applizieren eines Punktionssystems wie z.B. eines zentralvenösen Katheters anzugeben.

Die zuvor genannte Aufgabe wird durch ein Punktionssystem gemäß Anspruch 1 gelöst.Die Erfindung hat den Vorteil, dass der zentralvenöse Katheter wesentlich einfacher und schneller am Patienten appliziert werden kann als bekannte Lösungen. Hierbei kann auf den Seldinger-Draht und die damit verbundenen, relativ zeitaufwendigen Schritte vollständig verzichtet werden. Die Erfindung greift dabei auf die durchaus vorteilhaften Grundprinzipien der Seldinger-Technik zurück, entwickelt diese aber weiter, so dass die Risiken für den Patienten minimiert werden. Dadurch, dass der Katheter einen mehrschal igen Aufbau mit einer innen angeordneten Punktionsnadel, einem außen darum angeordneten Katheterschlauch und einer dazwischenliegenden Schicht in Form der Kanüle aufweist, und wobei die einzelnen Schichten Punktionsnadel/Kanüle/Katheterschlauch bzw. dessen Arbeitsvolumen im Prinzip beliebig gegeneinander längsverschieblich sind, wird eine ganz neue Technik des Applizierens eines zentralvenösen Katheters ermöglicht. Hierbei kann die Kanüle nach entsprechender Punktion einer Vene mittels des Punktionsabschnitts der Punktionsnadel in die so gebildete Öffnung in die Vene eingeschoben werden. Dabei bewirkt die Kanüle bereits einen Schutz der Vene vor Verletzungen durch die Spitze der Punktionsnadel. Zudem fungiert die Kanüle danach als Einführhilfe des Katheterschlauchs in die Vene, da sie in diesem Zustand eine ähnliche Funktion hat wie bisher der Seldinger-Draht. Im Unterschied zum Seldinger-Draht kann die Kanüle hinsichtlich ihrer Lage aber immer kontrolliert werden und zu einem gewünschten Zeitpunkt auch entfernt werden. Ebenso kann die Punktionsnadel zu einem gewünschten Zeitpunkt zurückgezogen oder ganz entfernt werden.

Ein weiterer Vorteil der Erfindung ist, dass in Fällen, in denen bisher mehrere periphervenöse Venenzugänge geschaffen wurden, nun ein einziger Zugang durch den erfindungsgemäßen zentralvenösen Katheter möglich und ausreichend ist. Aufgrund der Komplexität, die mit dem Anlegen zentralvenöser Katheter gemäß dem Stand der Technik verbunden ist, hat man bisher in manchen Fällen statt eines zentralvenösen Katheters mehrere periphervenöse Venenzugänge angelegt. Auch solche nachteiligen Situationen können nun durch die hier beschriebene Erfindung verbessert werden. Hierdurch kann die Infektionsgefahr verringert werden. Die bisher aufwendige Pflege mehrerer Venenzugänge wird vereinfacht, da nur noch der eine zentralvenöse Zugang erforderlich ist. Auch die Übersichtlichkeit bei der Medikamentenapplikation und somit die Patientensicherheit wird erhöht.

Der erfindungsgemäße zentralvenöse Katheter kann in verschiedenen Größen bereitgestellt werden, z.B. für unterschiedlich große Menschen (Erwachsene/Kinder).

Es wurde ferner erkannt, dass das zuvor anhand des Beispiels eines zentralvenösen Katheters beschriebene Punktionssystem auch anderweitig für Punktionen an Körperteilen vorteilhaft eingesetzt werden kann. Die beschriebenen Teile des Punktionssystems bzw. des zentralvenösen Katheters bleiben dabei wie zuvor beschrieben, sind jedoch nach Anwendungsfall insbesondere hinsichtlich ihrer Länge und gegebenenfalls des Durchmessers anzupassen.

Dementsprechend wird die eingangs genannte Aufgabe auch durch ein allgemeines Punktionssystem gemäß Anspruch 1 gelöst. Im Vergleich mit dem zuvor beschriebenen Anwendungsfall eines zentralvenösen Katheters (oder allgemein Katheter) weist das Punktionssystem statt eines Katheterschlauches einen äußeren tubusförmigen Körper auf, der starr oder flexibel sein kann, z.B. in Form eines Rohrs oder Schlauches. Ferner weist das Punktionssystem statt einer Kanüle einen inneren tubusförmigen Körper auf. Dieser kann auch als Kanüle ausgebildet sein, was aber nicht zwingend ist. Mit einem solchen Punktionssystem kann, im Vergleich zum zentralvenösen Katheter, nicht nur eine Vene punktiert werden, sondern grundsätzlich jedes Körperteil eines Lebewesens.

Dementsprechend kann als zu punktierendes Körperteil eines Lebewesens jeder Hohlkörper mit dem Punktionssystem vorteilhaft punktiert werden, wie z.B. Blase, Luftröhre oder Gefäße jeder Art, insbesondere Venen oder Arterien. Ferner können auch Nichthohlkörper vorteilhaft punktiert werden, z.B. Organe, Flüssigkeitsansammlungen, wie z.B. Abszesse. Auch Organzwischenräume können vorteilhaft punktiert werden.

Auf diese Weise können mit dem Punktionssystem folgende Anwendungsgebiete abgedeckt werden: Angiologie, Kardiologie, Punktion des Pleurazwischenraumes (Flüssigkeiten, Luft), Urologie, (Interventionelle) Radiologie, Tracheotomie und Koniotomie, Implantation von Herzunterstützungssystemen.

Es ist dabei vorteilhaft, wenn das Punktionssystem oder zumindest der äußere tubusförmige Körper transparent ist. Dies erlaubt eine verbesserte Beurteilbarkeit des Status des Punktionsvorganges, u.a. durch Farbe und Flussgeschwindigkeit der in das Punktionssystem strömenden Flüssigkeiten.

In diesem Sinne ist die nachfolgende Beschreibung, die sich am Beispiel des zentralvenösen Katheters (oder nur Katheter) orientiert, auch in dem Sinne zu verstehen, dass die dort beschriebenen Anwendungen und Funktionen sowie die technischen Merkmale auch durch das Punktionssystem im allgemeinen Sinne realisiert werden können. In diesem Sinne ist die nachfolgende Beschreibung auch auf das Punktionssystem im allgemeinen bezogen, somit ist nachfolgend mit Katheterschlauch auch allgemein der äußere tubusförmige Körper gemeint, mit Kanüle der innere tubusförmige Körper und mit Vene der zuvor erläuterte, zu punktierende Körperteil eines Lebewesens.

Das erfindungsgemäße Punktionssystem kann auch in Kombination mit einem Seldinger-Draht eingesetzt werden. Hierbei ist es vorteilhaft, wenn in diesem Fall ein hohler Seldinger-Draht eingesetzt wird, der hinsichtlich der Länge wie ein konventioneller Seldinger-Draht oder in kürzerer Version ausgebildet sein kann. Durch die Gestaltung als hohler Seldinger-Draht wird es möglich, eine Aspiration von Blut, Flüssigkeiten, Luft zu gestatten, somit kann die Lage des hohlen Seldinger-Drahts im Bereich der Zielstruktur verifiziert werden, bevor der Punktionskanal aufgeweitet wird. Zudem kann im Notfall auch bereits ein Medikament über den eingeführten Seldinger-Draht vor Einbringen weiterer Elemente des Punktionssystems injiziert werden, was eine wichtige Zeitersparnis ermöglicht. Der hohle Seldinger-Draht kann nach proximal, d.h. zum Benutzer hin, kompatibel zu einem Aspirationshilfsmittel sein, z.B. einer Spritze, ggf. über einen Adapter, der sich bereits auf der Spritze befindet. Dies hat den Vorteil, dass zunächst ein spontaner Rückfluss über den hohlen Seldinger-Draht abgewartet werden kann (pulsierend z.B. bei einer arteriellen Fehlpunktion) und dann das Aspirationshilfsmittel aufgesteckt werden kann. Vor Einführung des hohlen Seldinger-Drahts ist zunächst die Punktionsnadel über den Draht zurückzuziehen.

Das erfindungsgemäße Punktionssystem und insbesondere dessen äußerer tubusförmiger Körper ist zum Verweilen in einem Körperteil eines Lebewesens eingerichtet. Als "Verweilen" gilt dabei das Verweilen im üblichen medizinischen Sinne, wie es beispielsweise bei einem ZVK gilt, d.h. ein Verweilen im Bereich von Tagen oder Wochen oder Monaten. Das Punktionssystem und insbesondere dessen äußerer tubusförmiger Körper muss dabei nicht von einem Anwender in der Position gehalten werden, sondern ist auch in dem Sinne zum Verweilen eingerichtet, dass es beim Verweilen den menschlichen Körper nicht schädigen kann. Hierfür ist der äußere tubusförmige Körper beispielsweise aus einem thermoplastischen, stumpfen, nicht abscherbaren Material ausgebildet. Dementsprechend kann der äußere tubusförmige Körper als Schlauch oder Röhrchen ausgebildet sein. Nach Anbringung des äußeren tubusförmigen Körpers an einem Körperteil eines Lebewesens mittels des Punktionssystems werden der innere tubusförmige Körper sowie die Punktionsnadel vollständig aus dem äußeren tubusförmigen Körper entfernt, sodass während der Phase des Verweilens in dem Körperteil nur der äußere tubusförmige Körper dort verweilt. Aufgrund der günstig gewählten Durchmesserverhältnisse zwischen äußeren tubusförmigen Körper, innerem tubusförmigen Körper und Punktionsnadel ist die Anbringung des Punktionssystems in einem Körperteil eines Lebewesens sehr einfach möglich, insbesondere ohne einen Hautschnitt.

Zur Fixierung des Punktionssystems und insbesondere dessen äußeren tubusförmigen Körpers am Körperteil eines Lebewesens, z.B. an der Haut, kann z.B. ein Ankleben mittels eines Pflasters oder ein Festnähen an der Haut erfolgen. In einer vorteilhaften Weiterbildung der Erfindung weist das Punktionssystem einen Click-Clip-Mechanismus zur Fixierung an der Haut auf. Durch einen solchen Click-Clip-Mechanismus, der ähnlich wie bei Klammern zum Wundverschluss ausgebildet sein kann, können durch manuelle Betätigung, z.B. Fingerdruck von oben, Fixierungsklammern in die Haut gebohrt werden, sodass das Punktionssystem sicher an der Haut fixiert ist. Ein zeitaufwändiges Annähen des Punktionssystems kann dann entfallen.

Im Hinblick auf Jurisdiktionen, in denen Behandlungsverfahren dem Patentschutz zugänglich sind, wird die eingangs genannte Aufgabe außerdem gelöst durch ein Verfahren zum Applizieren eines zentralvenösen Katheters an einem Patienten, insbesondere eines Katheters der zuvor erläuterten Art, mit folgenden Schritten:
a) Punktion der Vene des Patienten mittels eines aus einem patientennahen Ende des Katheters herausragenden Punktionsabschnitts der Punktionsnadel,
b) Vorschieben der Kanüle in die Vene, d.h. über den Punktionsabschnitt der Punktionsnadel, wenn die Punktionsnadel korrekt in der Vene platziert ist,
c) Vorschieben des Katheterschlauchs über die in der Vene platzierte Kanüle, so dass auch der Katheterschlauch in die Vene eingeführt ist.

Die Punktionsnadel kann vor oder nach dem Einführen des Katheterschlauchs in die Vene zurückgezogen oder entfernt werden. Insbesondere ist ein Entfernen der Punktionsnadel vor dem Einbringen des Katheterschlauchs in die Vene vorteilhaft, um eventuelle Verletzungsgefahren weiter zu minimieren. Schließlich kann auch die Kanüle entfernt werden, indem diese aus dem Katheterschlauch am patientenfernen Ende herausgezogen wird. In gleicher Weise kann die Punktionsnadel entfernt werden.

Hierbei wurden zunächst nur die wesentlichen Schritte genannt. Weitere in der Praxis sinnvolle und erforderliche Schritte, wie z.B. die Aspiration von venösem Blut zur Kontrolle der Lage der Punktionsnadel, Fixierung der Komponenten des Katheters und ähnliches sind ebenfalls auszuführen. Ein beispielhafter Vorgang des Applizierens des zentralvenösen Katheters mit weiteren Details wird nachfolgend noch im Rahmen der Erläuterungen der Ausführungsbeispiele angegeben.

Gemäß einer vorteilhaften Weiterbildung der Erfindung ist eine Längsverschiebung des Katheterschlauchs gegenüber der Kanüle und eine Längsverschiebung der Kanüle gegenüber der Punktionsnadel jeweils unabhängig voneinander über Betätigungsmittel von einem patientenfernen Ende des Katheters steuerbar. Hierdurch wird die zuvor erwähnte, innovative Technik des Applizierens des Katheters weiter gefördert. Als Betätigungsmittel für die Längsverschiebung der Punktionsnadel kann z.B. eine Spritze dienen, die am patientenfernen Ende mit der Punktionsnadel verbunden ist. Als Betätigungsmittel der Kanüle kann z.B. eine Fixierungsfahne dienen, die am patientenfernen Ende des Katheters mit der Kanüle verbunden ist.

Gemäß einer vorteilhaften Weiterbildung der Erfindung weist der Katheter keine Dilatationskanüle auf. Dies hat den Vorteil, dass der Katheter einfach und mit wenig Komponenten aufgebaut sein kann und dementsprechend auch einfach zu bedienen ist. Insbesondere ist keine gesonderte Bedienung einer Dilatationskanüle erforderlich.

Gemäß einer vorteilhaften Weiterbildung der Erfindung weist der Katheter zum Einführen des Katheterschlauchs in die Vene am patientennahen Ende einen rampenförmig ansteigenden Dilatationskörper auf, durch den die Kanüle hindurchgeführt ist. Dies hat den Vorteil, dass die für die Einführung des Katheterschlauchs in die Vene notwendige Dilatation auf einfache Weise durchgeführt werden kann, indem der Dilatationskörper zunächst über die Kanüle in die auf diese Weise gebildete Körperöffnung geschoben wird. Hierfür ist der Dilatationskörper vorteilhafterweise gegenüber der Kanüle längsverschieblich ausgebildet. Der Dilatationskörper kann dabei durch Kraftbeaufschlagung beim Vorschieben des Katheterschlauchs durch diesen gegenüber der Kanüle vorangeschoben werden. Gemäß einer vorteilhaften Weiterbildung der Erfindung ist der Dilatationskörper am Katheterschlauch befestigt oder Teil des Katheterschlauchs. Durch eine solche feste Verbindung zwischen Dilatationskörper und Katheterschlauch ist die Lage des Dilatationskörpers auch jederzeit zu kontrollieren. So kann der Dilatationskörper z.B. als am patientennahen Ende des Katheterschlauchs rampenförmig ausgebildeter Bereich des Katheterschlauchs ausgestaltet sein.

Der rampenförmig ansteigende Teil des Dilatationskörpers kann linear oder nicht linear rampenförmig ansteigend ausgebildet sein. Es ist ferner vorteilhaft, wenn der Dilatationskörper aus einem Material mit geringem Reibungswiderstand, d.h. einem besonders gleitfähigen Material, ausgebildet ist oder zumindest mit einem solchen Material beschichtet ist. Auch hierdurch kann der Reibungswiderstand beim Vorschieben durch die Haut und somit beim Dilatationsvorgang minimiert werden.

Wie erwähnt, ist nach erfolgter Punktion eines Körperteils mittels eines aus dem patientennahen Ende des Punktionssystems herausragenden

Punktionsabschnitts der Punktionsnadel der innere tubusförmige Körper aus dem patientennahen Ende des Punktionssystems zumindest teilweise aus dem äußeren tubusförmigen Körper herausschiebbar. Der innere tubusförmige Körper ist dabei somit zum Patienten hin vorschiebbar und kann dabei ohne Weiteres auch in das Körperteil des Lebewesens vorgeschoben werden und dabei die Haut durchdringen. Auch der äußere tubusförmige Körper kann distal zum Patienten hin über den inneren tubusförmigen Körper geschoben werden und kann dabei ohne Weiteres auch in das Körperteil des Lebewesens vorgeschoben werden und dabei die Haut durchdringen, insbesondere wenn die Punktionsnadel entfernt oder zurückgezogen ist.

Gemäß einer vorteilhaften Weiterbildung der Erfindung ist der Katheterschlauch zumindest außerhalb der Vene von einer ersten Umhüllung umgeben, durch die der Katheterschlauch außerhalb der Vene steril gehalten ist. Beim Einschieben des Katheterschlauchs in die Vene wird die erste Umhüllung in Folge des Hautkontakts am Patienten zurückgeschoben, so dass der Katheterschlauch im sterilen Zustand in die Vene gelangt. Dies ist für den Anwender mit keinem zusätzlichen Aufwand verbunden, da sich die erste Umhüllung beim Einschieben des Katheterschlauchs in die Vene automatisch zurückschiebt bzw. zusammenschiebt. Die erste Umhüllung kann z.B. in Form einer beutelartigen Hülle oder eines Faltenbalgs ausgebildet sein.

Eine weitere Besonderheit des erfindungsgemäßen Katheters besteht darin, dass eine kleinlumige Kanüle verwendet werden kann. Dies hat den Vorteil, dass die durch die Punktionsnadel geschaffene Hautöffnung beim Durchschieben der Kanüle nicht wesentlich geweitet werden muss. Insbesondere ist hierbei keine Dilatation im üblichen medizinischen Sinne erforderlich. Daher kann der erfindungsgemäße Katheter auch ohne Dilatationskanüle realisiert werden. Stattdessen hat die Kanüle die Funktion einer Sicherung des durch die Punktionsnadel geschaffenen Lumens. Sie kann daher auch als Lumensicherungskanüle bezeichnet werden. Gemäß der Erfindung ist der Außendurchmesser der Kanüle daher maximal doppelt so groß wie der Außendurchmesser der Punktionsnadel. In einer vorteilhaften Weiterbildung ist der Außendurchmesser der Kanüle maximal 1,5 mal so groß ist wie der Außendurchmesser der Punktionsnadel.

Im Unterschied zu anderen, bekannten Vorschlägen wird somit eine Punktionsnadel vorgeschlagen, die einen geringeren Durchmesser hat als der Katheterschlauch und somit nur ein kleines Loch in der Haut hinterlässt.

Hierin liegt auch ein entscheidender Vorteil gegenüber bekannten Katheter-Lösungen. Bei bekannten Systemen wird z.B. eine Kanüle mit einem großen Durchmesser verwendet, durch die dann der Katheterschlauch in die Vene eingeführt wird. Die Kanüle kann belassen oder ggf. gespalten und entfernt werden. Das Punktionsloch in der Haut weist bei solchen Systemen einen größeren Durchmesser als der Katheterschlauch auf, wodurch es in der Regel zu Blutungen aus der Hauteintrittsstelle des Katheterschlauchs kommt. Zudem wird der Durchmesser des einzuführenden Katheterschlauchs durch den Durchmesser der Kanüle begrenzt. Da diese bereits einen gewissen Durchmesser haben muss, ist der Durchmesser des Katheterschlauchs begrenzt, so dass keine besonders hohen Flüssigkeitsmengen pro Zeiteinheit in die Vene infundiert werden können. Nachteilig bei bekannten Systeme ist ferner, dass die zur Punktion verwendete Punktionsnadel einen ebenso großen Durchmesser hat, so dass die Punktion traumatisierend ist. Fehlpunktionen können erhebliche Verletzungen zur Folge haben. Solche Nachteile werden durch die vorliegende Erfindung überwunden.

Dementsprechend ist im Übergang von der Punktionsnadel zur Kanüle nur ein geringer Kalibersprung vorhanden. Um das Einführen der Kanüle noch weiter zu vereinfachen, kann diese am patientennahen Ende abgerundet ausgebildet sein.

Hinsichtlich des Katheterschlauchs ergibt sich im Vergleich zur Kanüle ein grö-ßerer Kalibersprung, was notwendig ist, um auch einen erforderlichen Innendurchmesser zu realisieren, der für die Zuführung von Flüssigkeiten in hoher Menge notwendig ist. Gemäß der Erfindung ist daher der Außendurchmesser des Katheterschlauchs wenigstens doppelt so groß wie der Außendurchmesser der Kanüle. Hierdurch werden große Durchflussmengen gewährleistet. Gemäß einer vorteilhaften Weiterbildung der Erfindung ist der Außendurchmesser des Katheterschlauchs wenigstens dreimal so groß wie der Außendurchmesser der Kanüle.

Dementsprechend ist erst beim Einführen des Katheterschlauchs in die Vene ein Dilatationsschritt erforderlich.

Gemäß einer vorteilhaften Weiterbildung der Erfindung weist der Katheter am patientenfernen Ende ein Aspirationshilfsmittel oder einen Aspirationsanschluss zum Anschluss eines Aspirationshilfsmittels auf. Das Aspirationshilfsmittel, das z.B. als konventionelle Spritze ausgebildet sein kann, kann über den Aspirationsanschluss z.B. mit der Punktionsnadel verbunden werden. In diesem Fall ist es vorteilhaft, die Punktionsnadel als Hohlnadel auszubilden, so dass durch die Punktionsnadel hindurch Flüssigkeiten transportiert werden können. Das Aspirationshilfsmittel oder ein weiteres Aspirationshilfsmittel kann auch am patientenfernen Ende mit der Kanüle verbunden werden. Auf diese Weise kann auch die Kanüle am patientenfernen Ende mit einem Aspirationshilfsmittel oder auch anderen Systemen, z.B. Schlauchsystemen, kompatibel sein, was zur Differenzierung zwischen venöser und arterieller Punktion sehr hilfreich ist. Hierin ist ein weiterer wesentlicher Vorteil der Erfindung zu sehen. Der Benutzer kann z.B. einen Infusionsschlauch anschließen und visuell feststellen, ob das Blut pulsierend in diesen Infusionsschlauch aufsteigt (arterielle Punktion) oder nicht. Außerdem kann man auch bei Patienten mit einem nicht-pulsatilen Blutfluss, z.B. bei implantierten Herzunterstützungssystemen, leichter zwischen venöser und arterieller Punktion differenzieren.

Gemäß einer vorteilhaften Weiterbildung der Erfindung ist der Katheterschlauch zwischen der Spitze der Punktionsnadel und dem Aspirationsanschluss oder dem Aspirationshilfsmittel angeordnet.

Gemäß einer vorteilhaften Weiterbildung der Erfindung weist der Katheterschlauch eine Länge von wenigstens 12 cm auf, er kann für viele Anwendungen aber auch länger ausgebildet sein.

Gemäß einer vorteilhaften Weiterbildung der Erfindung ist der Katheterschlauch mehrlumig ausgebildet. Dies hat den Vorteil, dass außer dem Arbeitslumen noch ein oder mehrere Lumen zur Verfügung stehen, über die separat z.B. Flüssigkeiten transportiert werden können oder eine EKG-Ableitung erfolgen kann. So kann der Katheter z.B. mit einem Arbeitslumen und einem oder mehreren Hilfslumen ausgebildet sein, z.B. mit seitlichen intravenösen Austrittsstellen. Gemäß einer vorteilhaften Weiterbildung der Erfindung sind einzelne Lumen entfernbar und/oder ergänzbar. Hierdurch wird ein modulares Lumensystem geschaffen, das es erlaubt, vorab oder bei bereits eingeführtem Katheterschlauch Lumen zu entfernen und/oder zu ergänzen, z.B. um die Infektionsgefahr bei längerem Verweilen des Katheterschlauchs im Patienten zu senken.

Gemäß einer vorteilhaften Weiterbildung der Erfindung besteht die Kanüle aus Kunststoffmaterial, insbesondere aus thermoplastischem Kunststoffmaterial. Hierdurch kann die Kanüle relativ flexibel gestaltet sein und ist dennoch robust. Mögliche Verletzungsrisiken werden dadurch minimiert. Auch der Katheterschlauch kann aus Kunststoffmaterial bestehen. Die Punktionsnadel kann vorteilhaft als Metallnadel ausgebildet sein.

Gemäß einer vorteilhaften Weiterbildung der Erfindung ist die Kanüle länger als der Katheterschlauch und/oder die Punktionsnadel länger als die Kanüle. Dementsprechend kann die Kanüle an beiden Seiten aus dem Katheterschlauch hervorstehen und die Punktionsnadel kann an beiden Seiten aus der Kanüle hervorstehen. Dies vereinfacht das Längsverschieben und Entfernen der Punktionsnadel und der Kanüle jeweils vom patientenfernen Ende des Katheters. Gemäß einer vorteilhaften Weiterbildung der Erfindung weist der Katheter eine zweite Umhüllung auf, die am patientenfernen Ende des Katheters angeordnet ist und zumindest den Bereich der Kanüle umgibt, der am patientenfernen Ende des Katheters aus dem Katheterschlauch hervorsteht, so dass die Kanüle in diesem Bereich steril gehalten ist. Die erste Umhüllung und die zweite Umhüllung können als separate Umhüllungen ausgebildet sein. Eine solche zweigeteilte Schutzhülle ist eine weitere Besonderheit des erfindungsgemäßen Katheters. Die zweite Umhüllung kann z.B. in Form einer beutelartigen Hülle oder eines Faltenbalgs ausgebildet sein.

Gemäß einer vorteilhaften Weiterbildung der Erfindung weist der Katheter an seiner gesamten Außenoberfläche und/oder Innenoberfläche oder Teilbereichen davon eine flüssigkeitsabweisende Oberfläche auf, z.B. eine Oberfläche in der Art eines Lotuseffekts. Auf diese Weise weist der Katheter in solchen Bereichen zugleich blutabweisende Eigenschaften auf. Dies hat den Vorteil, dass sich die Lumen nicht mehr mit Blut oder geronnenem Blut oder Sekret verstopfen können. Zudem wird der Katheter in hygienischer und infektiologischer Hinsicht verbessert. Außerdem ist der Katheter im Gebrauch angenehmer und ästhetischer, da auch von außen keine Blutauflagerungen und Verschmutzungen mehr auftreten. Die Oberfläche weist somit eine geringe Benetzbarkeit auf, Flüssigkeiten wie Wasser und Blutperlen tropfen ab und nehmen dabei auch Schmutzpartikel auf der Oberfläche mit.

Auf diese Weise wird ein neuartiger Katheter angegeben, der teleskop- und lamellenartige Eigenschaften besitzt. Aufgrund seines erfinderischen dreischichtigen Aufbaus stabilisiert sich der Katheter während des Punktionsvorgangs selbst. Aufgrund des zunehmenden Durchmessers der Schichten von innen nach außen erfolgt eine zunehmende Aufdehnung des Punktionskanals ohne größere Kalibersprünge. Die Punktionsnadel kann hierbei ähnlich dünn ausgebildet sein wie bei der Seldinger-Technik, um bei einer Fehlpunktion Gewebe und Gefäße möglichst wenig zu traumatisieren. Am patientenfernen Ende des Katheters, hinter dem Katheterschlauch, kann eine Spritze zur Blutaspiration und Verifizierung der intravenösen Lage angeordnet werden. Unmittelbar nach Blutaspiration wird die Kanüle über die Punktionsnadel eine definierte Strecke in die Vene eingebracht. Dadurch wird die Gefahr der Dislokation des Punktionssystems aus der Vene heraus vermindert. Danach wird der Katheterschlauch über die Kanüle in den Körper vorgeschoben. Eine zurückschiebbare erste Umhüllung hält hierbei den Katheterschlauch steril. Dieser weist am patientennahen Ende einen Dilatationskörper, z.B. aus einem thermoplastischen Material, auf. Somit kann auf einem gesonderten Dilatator verzichtet werden. Im Gegensatz zur Seldinger-Technik sind daher deutlich weniger Arbeitsschritte erforderlich. Hierdurch wird die Verletzungs- und Infektionsgefahr für Patienten und Anwender minimiert. Es können keine durch einen Seldinger-Draht bedingten Komplikationen mehr auftreten. Die Lage des Katheterschlauchs kann mittels EKG-Ableitung kontinuierlich überwacht werden. Durch die einfache Handhabung bei geringeren Kosten werden neue Anwendungsgebiete, wie z.B. die präklinische Notfallmedizin, erschlossen.

Mit der ersten und/oder der zweiten Umhüllung kann zudem eine Art "abgeschlossenes System" geschaffen werden, dass in vollem Umfang die einschlägigen Hygieneaspekte erfüllen kann.

Die Erfindung kann z.B. als Punktionsset mit folgenden Bauteilen bereitgestellt werden (Längenangaben sind nur beispielhaft, in der Praxis variabel):
1) 25 cm lange Punktionsnadel 20G mit Schutzkappe über der Nadelspitze
2) 23,5 cm lange Kanüle z.B. in Form einer Kunststoffkanüle über der Punktionsnadel mit patientenferner Fixierungsfahne (Punktionsnadel und Kanüle befinden sich im Arbeitslumen des Katheterschlauchs)
3) 17 cm langer Katheterschlauch mit dilatierenden Eigenschaften (thermoplastisch), drei-lumig, davon ein kürzeres Arbeitslumen 14-16G und zwei Lumen zu je 12G mit seitlichen intravenösen Austrittsstellen, patientenfernen Verlängerungen ("Schenkel") und vormontierten Drei-Wege-Hähnen; zusätzlich Klemmen und Flügel zum Annähen
4) Kappe zum Verschluss des Arbeitslumens incl. der Kanüle, zusätzlicher Dreiwegehahn für mögliche Nutzung des Arbeitslumen als zusätzliches Infusionslumen nach Punktionsende
5) 10 ml-Spritze zur Blutaspiration (vorgefüllt mit 5ml Kochsalzlösung/destilliertem Wasser)
6) Zwei Spritzen zum Durchspülen der Schenkel nach Punktion (vorgefüllt mit jeweils 10 ml Kochsalzlösung/destilliertem Wasser)
7) Sterile zurückschiebbare Hülle zur Katheterschlauchummantelung
8) Nahtset und Skalpell

Zusammengefasst ergeben durch die Erfindung sich folgende weitere Vorteile:
- Unmittelbar nach Blutaspiration kann eine Kanüle in die Vene eingebracht werden. Dies vermindert die Gefahr, wieder aus dem Gefäß "herauszurutschen". Die Punktionsnadel kann sofort entfernt werden, es sind keine weiteren Bewegungen bei im Körper einliegender Punktionsnadel erforderlich. Dies minimiert die Verletzungsgefahr für den Patienten und den Anwender. Das Einbringen der Kanüle ist vergleichbar wenig traumatisch wie die Punktion selber, im Falle einer arteriellen Fehlpunktion ist das Komplikationsrisiko kalkulierbar.
- Die zurückschiebbare Umhüllung hält den Katheterschlauch steril, ein steriles Umfeld wie oben beschrieben ist nicht erforderlich. Der Platzbedarf ist minimal und ergibt sich lediglich aus der Summe des Bedarfs des Punktierenden und des Sets selbst.
- Die Gefahr von akzidentiellen Kontakten der sterilen Materialien mit der Umgebung wird dadurch minimiert, dass die Komponenten in einem Punktionssystem, nämlich dem erfindungsgemäßen Katheter, integriert sind.

Dies vermindert die Infektionsgefahr für Patient und Anwender.
- Es werden kein Seldinger-Draht oder andere Komponenten benötigt, die akzidentiell im Körper verbleiben oder andere Komplikationen verursachen könnten.
- Beim Vorschieben des Katheterschlauches kann eine permanente Lagekontrolle erfolgen, das EKG-Signal kann auf Wunsch direkt über die Spitze des Katheterschlauches abgeleitet werden. Zudem kann die venöse Lage des Katheterschlauches durch Blutaspiration über denselben zu jedem Zeitpunkt verifiziert werden ("Permanente Lumenkontrolle").
- Viele Schritte der klassischen Seldinger-Technik sind nicht mehr nötig (Dekonnektionen, Auffädelungen etc.), eine Assistenzperson ist nicht erforderlich.
- Die Braunüle^{®} ist den Anwendern bereits gut vertraut. Dies kann insbesondere erfahrenen Anwendern dabei helfen, die neue Punktionstechnik mit dem erfindungsgemäßen Katheter zu erlernen.
- Das System ist bei der Punktion trotz der langen Punktionsnadel ausreichend stabil und stabilisiert sich aufgrund der lamellenartigen Eigenschaft selbst.
- Der Dilatator ist in den Katheterschlauch integriert und besitzt thermoplastische Eigenschaften. Die Spitze des Katheterschlauches kann vergleichsweise weich gestaltet sein, da sie nur das bereits vorhandene Punktionsloch nutzen muss. In dem Punktionsloch befindet sich zum Zeitpunkt des Vorschiebens des Katheterschlauchs noch die Kanüle. Erst durch die Dilatationskörper erfolgt eine veritable "sanfte Dilatation" des Hautkanals. Eine solche "sanfte Dilatation" kann vorteilhaft durch einen rampenförmig ansteigenden Dilatationskörper gefördert werden, dessen Durchmesser auch nur "sanft" ansteigt, d.h. mit geringer Steigung.
- Die Spitze des Katheterschlauches kann aus einem weichen und somit gewebeschonenden Material bestehen, da eine Dilatation des Hautkanals und der Venenwand erst durch den nachfolgenden Dilatationskörper erfolgt. Da dieser Dilatationskörper der weichen Spitze nur folgt, geht bei Lage des Dilatationskörpers im Gewebe bzw. in der Vene nur eine sehr geringe Verletzungsgefahr von diesem aus. Die Verletzungsgefahr wird durch die eben beschriebenen thermoplastischen Eigenschaften des Dilatationskörpers weiter verringert. Der Dilatationskörper kann eine geringe "Anstiegssteilheit" aufweisen und somit eine sehr "sanfte" Dilatation ermöglichen.
- Die Technik kann im präklinischen Bereich zur Anwendung kommen.
- Erhöhte Kosten am Anfang der Entwicklung können durch Einsparungen an anderer Stelle ausgeglichen werden (geringer Zeitaufwand, Minimierung der Gefahr des Herunterfallens von sterilem Material mit notwendigem Ersatz während der Prozedur etc.).

Mit dem neuen Punktionssystem ist die Punktion jeder größeren Vene möglich, auch wenn primär nicht zwingend eine zentralvenöse Lage der Spitze des Katheterschlauchs erforderlich ist. Diese kann jedoch problemlos erreicht werden. Aufgrund der Möglichkeit der Anlage eines mehrlumigen Systems mit hohen Durchflussraten ohne die Notwendigkeit der Anlage eines sterilen Umfelds ergeben sich z.B. folgende Anwendungsmöglichkeiten:
- Präklinische Notfallmedizin mit Schwerpunkt Traumaversorgung
- Innerklinische Notfallmedizin
- Intensivmedizin
- Dialyse, z.B. auf Intensivstationen
- Anästhesiologie mit Schwerpunkt auf große chirurgische Eingriffe mit bedeutsamen Volumenverlusten
- Medizin in Kriegs-, Krisen- und Katastrophengebieten
- Medizin in räumlich beengten Verhältnissen (Flugzeugen, bodengebundenen Schutz- und Rettungsfahrzeugen, Schiffen etc.)

Das neue Punktionssystem besitzt teleskop- und lamellenartige Eigenschaften. Es erfolgt die zweifache Durchführung einer Art Catheter-Over-Needle-Technik direkt hintereinander an einem Punktionssystem. Hierdurch ergibt sich eine komplett neue Punktionstechnik. Der Punktionskanal ist zunächst klein, wodurch das Risiko von Komplikationen aufgrund einer Fehlpunktion überschaubar ist. Dieser Vorteil war bis dato der Seldinger-Technik vorbehalten, da nur sie es erlaubte, über verschiedene Zwischenschritte durch einen kleinen Punktionskanal durch Dilatation desselben einen großen Katheterschlauch einzuführen.

Bei dem neuen Punktionssystem befindet sich der Katheterschlauch z.B. zwischen der Spitze der Punktionsnadel und der Spritze zur Blutaspiration. Ein paar Millimeter proximal der Spitze des Katheterschlauches kann in diesem ein festerer Anteil eingebettet sein, dessen Länge ebenso nur einige Millimeter beträgt. Er hat dilatierende Eigenschaften und ersetzt somit den bei der Seldinger-Technik verwendeten Dilatator. Eine Verletzung der Venenwand ist sehr unwahrscheinlich, da der festere Anteil selbst keine spitzen Eigenschaften hat und der weicheren Spitze des Katheterschlauches nur folgt. Zudem kann der dilatierende Anteil auch thermoplastische Eigenschaften haben, so dass er im Körper weicher wird und die umliegenden Strukturen nicht mehr relevant irritieren kann.

Dadurch, dass nach erfolgter Venenpunktion zunächst eine Kanüle einige Zentimeter in die Vene eingebracht und die Punktionsnadel sofort zurückgezogen oder entfernt werden kann, kann die intravenöse Lage vor Einbringen des Katheterschlauches effektiv gesichert werden. Beim Einbringen des Katheterschlauches besteht keine Verletzungsgefahr mehr durch eine einliegende spitze Punktionsnadel. Dadurch wird die Patientensicherheit verbessert. Die Verletzungsgefahr für den Anwender wird ebenso minimiert, da alle Komponenten in einem Punktionssystem integriert sind und weniger Arbeitsschritte erforderlich sind. Dadurch vermindert sich auch die Infektionsgefahr für Patient und Anwender. Die zurückschiebbare Umhüllung hält den Katheterschlauch steril, ein steriles Umfeld wie bei der klassischen Seldinger-Technik ist nicht erforderlich. Dies begünstigt alle Arten der Anwendung, z.B. im präklinischen Bereich.

Die Gefahr der Verletzung z.B. des Herzens durch einen sehr weit eingebrachten Seldinger-Draht besteht ebenfalls nicht mehr; zudem ist die Wahrscheinlichkeit von Herzrhythmusstörungen sehr gering, da sich die Kanüle lediglich so weit in der Vene befindet, um die intravenöse Lage zu sichern und dem nachfolgenden Katheterschlauch den Weg in die Vene zu bahnen. Im Vergleich zur Seldinger-Technik wird der Katheterschlauch nicht mehr direkt über einen Draht bis vor das Herz geführt. Das erscheint auch nicht notwendig, wenn der Katheterschlauch, geführt von der Kanüle, sicher intravenös liegt und die "richtige Richtung" (zum Herzen hin) eingeschlagen hat. Dann kann er sicher in Richtung Herz vorgeschoben werden. Beachtet werden muss, dass bei der Seldinger-Technik Fehllagen häufig sind und falsche Wege mit Hilfe des Drahtes dann auch sehr konsequent verfolgt werden. Bereits beim Vorschieben des Katheterschlauches kann bei der Erfindung nun eine kontinuierliche EKG-Ableitung zur Lagekontrolle erfolgen. Die bei Einbringen des Seldinger-Drahtes bestehende Lageunsicherheit desselben bei der Seldinger-Technik ist somit nicht mehr gegeben.

Die Erfindung erlaubt eine erneute Kontrolle der venösen Lage nach Einbringen der Kanüle noch vor Einbringen des Katheterschlauches incl. des dilatierenden Anteils. Dadurch kann erneut eine arterielle Fehlpunktion/extravasale Lage des Systems erkannt werden, bevor eine Dilatation erfolgt. Das Risiko von Komplikationen wird hierdurch weiter vermindert. Sollte die zunächst eingebrachte Kanüle eine Fehllage aufweisen, so ist das Risiko überschaubar, da sie noch kein großes Lumen aufweist. Eine arterielle Fehlpunktion kann ggf. auch ohne Aspiration bemerkt werden, da nach Entfernen der Punktionsnadel im Regelfall helles Blut aus der Kanüle herauspulsieren wird bzw. dieses trotz des langen Katheters zügig herausläuft. Die Kanüle kann nicht akzidentiell im Körper verbleiben, da sie mit einer proximalen Fahne konstruktionsbedingt am Eindringen in den Körper gehindert wird.

Viele Schritte der klassischen Seldinger-Technik sind nicht mehr nötig. Es kann auf Dekonnektions- und Rekonnektions-/Auffädelungsschritte verzichtet werden. Dies reduziert die Komplexität des Verfahrens, zudem können Personal- und Materialkosten eingespart werden.

Zusammengefasst kann mit dem neuen Punktionssystem ein großer Katheterschlauch über einen initial kleinen Punktionskanal direkt und sicher in eine herznahe Vene eingebracht werden. Verletzungs- und Infektionsgefahr für Patient und Anwender werden minimiert, Gefahren durch einen einliegenden Seldinger-Draht bestehen nicht mehr. Die Lage des Katheterschlauches kann kontinuierlich überwacht werden. Das neue Punktionssystem erlaubt eine einfache Handhabung, Kosten können eingespart werden.

Die Erfindung kann mit folgenden Merkmalen vorteilhaft weitergebildet werden:
- EKG-Ableitung zur Lagekontrolle des patientennahen Endes des Katheterschlauchs in vereinfachter Form über einen Monitor auf dem Brustkorb, welcher nur einfache EKG-Signale erfasst; dieses Verfahren ist überall anwendbar, auch im präklinischen und innerklinischen notfallmedizinischen Setting.
- Entfernbares Lumen/modulares/ergänzbares System, um die Infektionsgefahr bei längerer Liegedauer des zentralvenösen Katheters zu verringern.
- Halbautomatisches System, welches die Position des patientennahen Endes des Katheterschlauchs selbständig anzeigt.
- Elektrisch leitende, metallbedampfte Spritze und/oder Katheterschlauch mit elektrisch leitenden Eigenschaften zur EKG-Abteilung.
- Ampelsystem auf dem zentralvenösen Katheter (mehrfarbiger zentralvenöser Katheter) und auf der Set-Verpackung (1-2-3, rot-gelb-grün) zur vereinfachten Bedienbarkeit, auch für den Anfänger/Ungeübten.
- System zur automatischen Auswertung der Blutflüsse an dem patientennahen Ende des Katheterschlauchs ("Vollautomat").
- System zur Positionsbestimmung des patientennahen Endes des Katheterschlauchs mittels Stromstärkemessung, der zentralvenöse Katheter muss nicht mehr initial "zu tief" eingeführt werden.
- System zur kontinuierlichen oder intermittierenden Lagekontrolle des patientennahen Endes des Katheterschlauchs, auch für die Normalstation.
- Integriertes Dopplersystem zur Detektion von arterieller und venöser Lage und von venösen Luftembolien im Rahmen von neurochirurgischen Eingriffen in sitzender Position mit automatischer Absaugung der Luft über ein weiteres distales Loch bzw. über weitere zusätzliche Löcher.
- Sich selbst verschließendes Lumen, nachdem eine innenliegende Kanüle entfernt wurde; dadurch werden höhere Flussraten durch die anderen Lumen ermöglicht.
- Punktionsnadel, die am patientenfernen Anteil nicht hart ist. Dadurch kann die Punktionsnadel nicht zu weit in die Vene eingeführt werden.
- Punktionsnadel, die aus hartem Kunststoff besteht, beim Vorschieben der Punktionsnadel wird diese weich (bzw. auch dann, wenn die Fixierungsfahne zurückgezogen wird, wobei hier eine Verbindung zwischen der Punktionsnadel und der Kanüle besteht).
- Mit der Spitze über einen Konnektor verbundene Punktionsnadel, der Katheterschlauch ist schlaufenähnlich aufgewickelt.
- Fixierung des zentralvenösen Katheters erfolgt über einen speziellen "Click-Mechanismus" mit Klammern an der Haut, ein Annähen ist nicht mehr erforderlich.
- Gleitmittel in der patientennahen (ersten) Umhüllung. Hierdurch kann der Katheterschlauch leichter durch die Haut und in die Vene geführt werden.

Die Erfindung wird nachfolgend anhand von Ausführungsbeispielen unter Verwendung von Zeichnungen näher erläutert.

Es zeigen
- Figur 1: einen zentralvenösen Katheter in einer ersten Ausführungsform und
- Figur 2: eine vergrößerte Detaildarstellung des patientennahen Endes des Katheters gemäß Figur 1 und
- Figur 3: eine zweite Ausführungsform eines zentralvenösen Katheters.

In den Figuren werden gleiche Bezugszeichen für einander entsprechende Elemente verwendet.

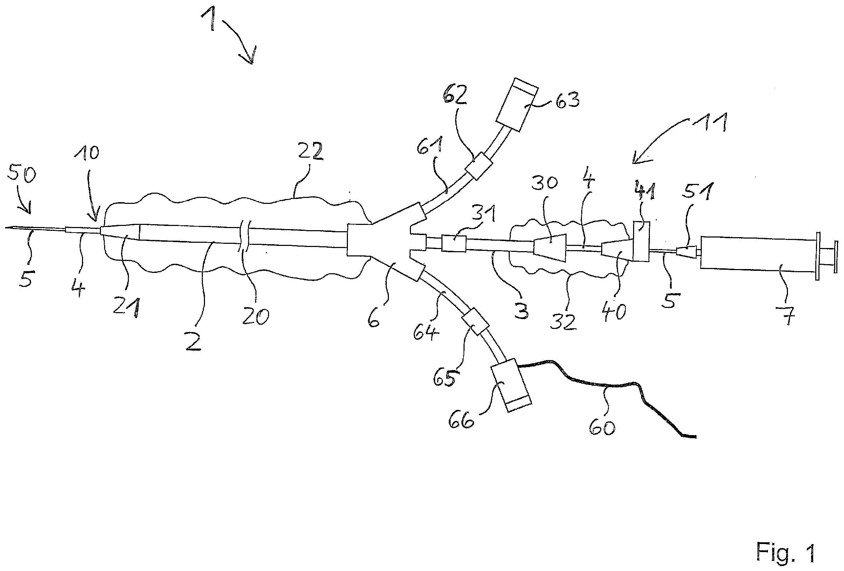

Die Figur 1 zeigt den grundsätzlichen Aufbau des erfindungsgemäßen Katheters 1. Es sei angenommen, dass im Auslieferungszustand alle Lumen bereits mit sterilem Kochsalz und/oder destilliertem Wasser vorgefüllt sind, damit der Katheter 1 ohne Entlüften direkt verwendet werden kann. Der Katheter 1 bzw. auch weitere Bestandteile, die das Punktionssystem bilden, sind in der dargestellten Form steril verpackt auszuliefern und nach dem Öffnen der Verpackung sofort ohne weitere Vorbereitung einsetzbar. Ein Öffnen unter sterilen Bedingungen ist nicht erforderlich.

Der Katheter 1 weist ein patientennahes Ende 10 auf, an dem Teile des Katheters 1 in eine Vene eines Patienten einzuführen sind. Der Katheter 1 weist ferner ein patientenfernes Ende 11 auf, das wie erkennbar am vom patientennahen Ende 10 abgewandten Endbereich des Katheters 1 angeordnet ist. Am patientenfernen Ende 11 erfolgt die Bedienung des Katheters 1 durch einen Anwender.

Der Katheter 1 weist einen Katheterschlauch 2, ein Arbeitslumen 3, eine Kanüle 4 sowie eine Punktionsnadel 5 auf. Innerhalb des Arbeitslumens 3 des Katheterschlauches 2 ist die Kanüle 4 längs durch den Katheterschlauch 2 bzw. das Arbeitslumen 3 hindurchgeführt. Innerhalb der Kanüle 4 ist die Punktionsnadel 5 längs durch die Kanüle 4 hindurchgeführt. Am patientennahen Ende 10 ragt die Punktionsnadel 5 mit einem Punktionsabschnitt 50 aus dem Katheter 1 heraus. Die Figur 1 zeigt dabei, dass die Kanüle 4 bereits aus dem Katheterschlauch 2 herausgeschoben ist, was zunächst, d.h. im Auslieferungszustand des Katheters 1, nicht der Fall ist. Dieser Fall tritt erst im Verlaufe des Applizierens des Katheters 1 an einem Patienten ein, wie nachfolgend noch erläutert wird. Zunächst ragt nur der Punktionsabschnitt 50 der Punktionsnadel 5 am patientennahen Ende 10 aus dem Katheter 1 heraus. Der Punktionsabschnitt 50 ist zunächst, d.h. im Auslieferungszustand, z.B. mittels einer Schutzkappe, wie sie bei Spritzen-Kanülen verwendet wird, vor unsterilen Einflüssen geschützt.

Der Katheter 1 ist relativ lang ausgebildet, wobei in der Figur 1 der Katheter 1 im mittleren Bereich verkürzt wiedergegeben ist, was durch die Markierung 20 symbolisiert werden soll. Tatsächlich kann der Katheter 1 deutlich längere Proportionen aufweisen.

Der Katheter 1 ist mehrlumig ausgebildet. Er weist neben dem Arbeitslumen 3 noch ein erstes Hilfslumen 61 mit einem ersten Katheteransatz 63 sowie ein zweites Hilfslumen 64 mit einem zweiten Katheteransatz 66 auf. Das Arbeitslumen 3 sowie die Hilfslumen 61, 64 sind über einen Verbindungsabschnitt 6, der auch als Fixierflügel des Katheters zum Befestigen an einem Patienten genutzt werden kann, zusammengeführt und mit dem Katheterschlauch 2 gekoppelt. Die Hilfslumen 61, 64 sind konventionell wie bei bekannten zentralen Venenkathetern ausgebildet, z.B. in Form eines jeweiligen Schlauches 61, 64, einer Schlauchklemme 62, 65 zum Abklemmen des Schlauches 61, 64 und einem Anschlusskörper, auch Katheteransatz genannt, über den jeweils eine Spritze oder ein Schlauch an einem der Hilfslumen 61, 64 angeschlossen werden kann. Beispielsweise kann am zweiten Hilfslumen 64 eine elektrische Anschlussleitung 60 für eine diskrete kontinuierliche EKG-Ableitung über den Katheterschlauch vorhanden sein.

Der Katheterschlauch 2 weist, wie in der Figur 1 auch erkennbar ist, einen relativ großen Kalibersprung im Vergleich zur Kanüle 4 auf. Daher ist zum Einführen des Katheterschlauches 2 in die Vene ein Dilatationsschritt erforderlich. Zu diesem Zweck befindet sich am patientennahen Ende 10 am Ende des Katheterschlauches 2 ein Dilatationskörper 21, der in Form eines angeschrägten Endes des Katheterschlauches 2 ausgebildet sein kann.

Um den Katheterschlauch 2 vor dem Einführen in die Vene steril zu halten, ist dieser von einer ersten Umhüllung 22 in Form einer beutelartigen Hülle umgeben. Diese erstreckt sich vom patientennahen Ende 10 hin bis zu dem Verbindungsabschnitt 6. Am patientennahen Ende 10 ist die erste Umhüllung 22 verschieblich auf dem Katheterschlauch 2 angeordnet und schiebt sich beim Vorschieben des Katheterschlauches 2 in die Vene eines Patienten automatisch durch den Hautkontakt zurück.

Am patientenfernen Ende 11 ist auf dem Arbeitslumen 3 eine Schlauch klemme 31 angeordnet, über die das Arbeitslumen 3 abklemmbar ist, zumindest nachdem die Kanüle 4 und die Punktionsnadel 5 daraus entfernt wurden.

Das Arbeitslumen 3 endet am patientenfernen Ende 11 mit einem Abschlussstück 30. Dieses dient als Anschlag für das Vorschieben der Kanüle 4, um sicherzustellen, dass die Kanüle 4 nicht unerwünscht weit vorgeschoben werden kann. Die Kanüle 4 endet am patientenfernen Ende 11 mit einem Abschlussstück 40, an dem eine Fixierungsfahne 41 angeordnet ist. Die Fixierungsfahne 41 dient dazu, dass die mittlere Schicht, d.h. die Kanüle 4, nicht übermäßig weit in die Vene vorgeschoben werden kann. Dadurch soll vermieden werden, dass die Kanüle 4 aus Versehen im Körper verbleibt bzw. das Herz irritiert, wenn sie zu weit aus dem Katheterschlauch 2 vorsteht und der Katheterschlauch 2 Richtung Herzen vorgeschoben wird. Zusätzlich kann die Fixierungsfahne 41 zum manuellen Greifen beim Vorschieben oder Herausziehen der Kanüle 4 genutzt werden, sie fungiert daher auch zugleich als Betätigungsmittel der Kanüle 4. Die Kanüle 4 kann nach vorne hin in Richtung patientennahes Ende 10 soweit vorgeschoben werden, bis das Abschlussstück 40 zur Anlage an dem Abschlussstück 30 gelangt.

Der Katheter 1 weist eine zweite Umhüllung 32 in Form einer beutelartigen Hülle auf, die den offen liegenden Bereich der Kanüle 4 am patientenfernen Ende 11 umgibt, um diesen steril zu halten. Das erfindungsgemäße Punktionssystem kann somit mit einer zweigeteilten sterilen Umhüllung ausgebildet sein, nämlich der ersten Umhüllung 22, die sich am Punktionssystem distal zum Patienten hin befindet, und zusätzlich der zweiten Umhüllung 32, die sich proximal entfernter vom Patienten befindet, wo der innere tubusförmige Körper 4 vor der Punktion aus dem äußeren tubusförmigen Körper 2 herausragt. Hierbei kann insbesondere der patientennahe Teil der Umhüllung, d.h. die erste Umhüllung 22, ein steriles Gleitgel aufweisen, durch das die Reibung beim Vorschieben des äußeren tubusförmigen Körpers in das Körperteil des Lebewesens und insbesondere durch die Haut minimiert wird.

Die Punktionsnadel 5 tritt am patientenfernen Ende 11 aus der Kanüle 4 aus. Sie weist beispielsweise einen Anschlusskörper 51 zum Anschluss einer Spritze 7 auf. Die Spritze 7 oder der Anschlusskörper 51 kann zugleich als Betätigungsmittel für das Herausziehen der Punktionsnadel 5 nach erfolgtem Einführen der Kanüle 4 in die Vene genutzt werden. Mittels der Spritze 7 kann eine Blutaspiration durchgeführt werden.

Beim Applizieren des Katheters 1 gemäß Figur 1 an einem Patienten kann vorteilhaft wie folgt vorgegangen werden:
1) Punktion der Vene mit der Punktionsnadel.
2) Bei Aspiration von venösem Blut Vorschieben der Kanüle in die Vene; hierbei erfolgt das Vorschieben an der Fixierungsfahne, bis sie nicht mehr weiter vorgeschoben werden kann, also das Arbeitslumen erreicht ist (hier passt sie bewusst nicht hinein).
3) Entfernen der Punktionsnadel.
4) Sofortiges Verschließen der Kanüle oder erneute Kontrolle der korrekten intravenösen Lage mit einer Spritze
5) Vorschieben des Katheterschlauches über die Kanüle in die Vene.
6) Entfernen der Kanüle.
7) Ggf. Fixierung des Katheterschlauches an der Haut.

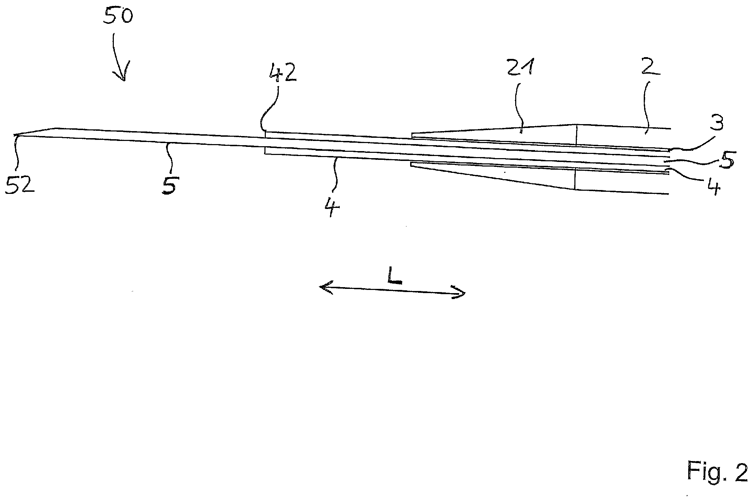

Die Figur 2 zeigt die Details des patientennahen Endes 10 des Katheters 1 in vergrößerter Schnittdarstellung. Erkennbar ist der erfinderische dreischichtige Aufbau, bei dem innerhalb des Katheterschlauches 2 bzw. innerhalb von dessen Arbeitslumen 3 die Kanüle 4 angeordnet ist, und innerhalb der Kanüle 4 die Punktionsnadel 5. Die Punktionsnadel 5 ist in Richtung der Längsachse L verschieblich, d.h. längsverschieblich. Die Kanüle 4 ist ebenfalls in Richtung der Längsachse L verschieblich, d.h. längsverschieblich. Das Arbeitslumen 3 kann dabei fest mit dem Katheterschlauch 2 verbunden sein. Die Kanüle 4 kann an ihrem patientennahen Ende 42 z.B. abgerundet ausgebildet sein, um das Einführen in die Vene zu erleichtern. Die Punktionsnadel 5 weist am Ende eine Spitze 52 auf, ähnlich wie bei Spritzen-Kanülen.

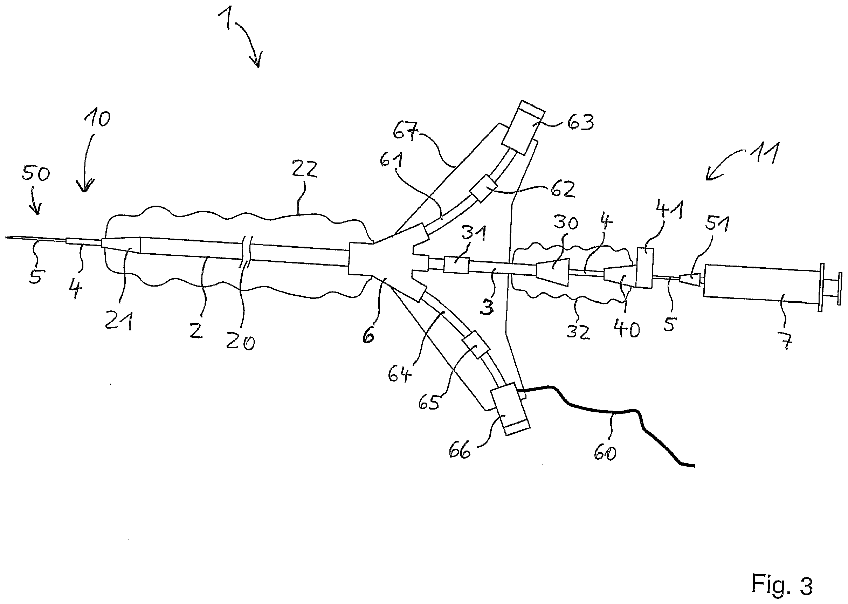

Die Figur 3 zeigt eine Ausführungsform des Katheters 1, bei der im Vergleich zur Ausführungsform der Figur 1 ein Haltemittel 67 ergänzt wurde, an dem die drei aus dem Verbindungsabschnitt 6 austretenden Schenkel, d.h. das Arbeitslumen 3 sowie die Hilfslumen 61, 64 sowie die damit verbundenen Teile, ganz oder teilweise fixiert sein können. Auf diese Weise wird die Handhabung vereinfacht, da diese Schenkel nicht mehr lose sich bewegen können, sondern in vordefinierten Positionen verbleiben. Das Haltemittel 67 kann z.B. in Form einer Halteplatte ausgebildet sein, z.B. in Dreiecksform oder ähnlicher Form.

Ergänzend kann am patientennahen Ende 10 des Katheters 1 ein Haltekörper angeordnet sein, der es dem Benutzer erlaubt, bei einer Punktion mit der zweiten Hand das patientennahe Ende 10 des Katheters 1 zu halten und zu steuern beziehungsweise weiter zu stabilisieren. Dieser Haltekörper kann am Katheter 1 gegenüber dem Katheterschlauch 2 verschieblich angebracht sein und kann so beim Einführen des Katheterschlauchs 2 in den Körper in einer festen Position gehalten werden. Zusätzlich kann der Haltekörper ähnlich wie bei einer marktüblichen peripheren Venenverweilkanüle Flügel enthalten, an denen der Katheter 1 zusätzlich über z.B. eine Naht fixiert werden kann. Der Haltekörper kann aber auch am Ende des Einführvorganges entfernt werden, d.h. lösbar am Katheter 1 befestigt sein.

## Patentansprüche

1. Punktionssystem (1) mit einem dreischichtigen Aufbau, bestehend aus einem äußeren tubusförmigen Körper (2), der zum Verweilen in einem Körperteil eines Lebewesens eingerichtet ist, einem inneren tubusförmigen Körper (4) und einer Punktionsnadel (5), wobei der innere tubusförmige Körper (4) durch ein Arbeitslumen (3) des äußeren tubusförmigen Körpers (2) geführt ist und gegenüber dem äußeren tubusförmigen Körper (2) längsverschieblich ist, und die Punktionsnadel (5) durch ein Punktionslumen des inneren tubusförmigen Körpers (4) geführt ist und der innere tubusförmige Körper (4) gegenüber der Punktionsnadel (5) längsverschieblich ist, wobei nach erfolgter Punktion eines Körperteils mittels eines aus einem patientennahen Ende (10) des Punktionssystems (1) herausragenden Punktionsabschnitts (50) der Punktionsnadel (5) der innere tubusförmige Körper (4) aus dem patientennahen Ende (10) des Punktionssystems (1) zumindest teilweise aus dem äußeren tubusförmigen Körper (2) herausschiebbar und dabei den aus dem patientennahen Ende (10) des Punktionssystems (1) herausragenden Punktionsabschnitt (50) der Punktionsnadel (5) wenigstens zum Teil in dem Punktionslumen des Inneren tubusförmigen Körpers (4) aufnehmend ausgebildet ist, **dadurch gekennzeichnet, dass** der Außendurchmesser des inneren tubusförmigen Körpers (4) maximal doppelt so groß ist wie der Außendurchmesser der Punktionsnadel (5) und dass der Außendurchmesser des äußeren tubusförmigen Körpers (2) wenigstens doppelt so groß ist wie der Außendurchmesser des inneren tubusförmigen Körpers (4).

2. Punktionssystem nach dem vorhergehenden Anspruch, **dadurch gekennzeichnet, dass** eine Längsverschiebung des äußeren tubusförmigen Körpers (2) gegenüber dem inneren tubusförmigen Körper (4) und eine Längsverschiebung des inneren tubusförmigen Körper (4) gegenüber der Punktionsnadel (5) jeweils unabhängig voneinander über Betätigungsmittel (40, 41, 51, 7) von einem patientenfernen Ende (11) des Punktionssystems (1) steuerbar ist.

3. Punktionssystem nach einem der vorhergehenden Ansprüche, **dadurch gekennzeichnet, dass** zum Einführen des äußeren tubusförmigen Körpers (2) in das Körperteil das Punktionssystem (1) am patientennahen Ende (10) einen rampenförmig ansteigenden Dilatationskörper (21) aufweist, durch den der innere tubusförmige Körper (4) hindurch geführt ist.

4. Punktionssystem nach dem vorhergehenden Anspruch, **dadurch gekennzeichnet, dass** der Dilatationskörper (21) gegenüber dem inneren tubusförmigen Körper (4) längsverschieblich ist.

5. Punktionssystem nach einem der Ansprüche 3 bis 4, **dadurch gekennzeichnet, dass** der Dilatationskörper (21) am äußeren tubusförmigen Körper (2) befestigt ist oder Teil des äußeren tubusförmigen Körpers (2) ist.

6. Punktionssystem nach einem der vorhergehenden Ansprüche, **dadurch gekennzeichnet, dass** der äußere tubusförmige Körper (2) zumindest außerhalb des Körperteils von einer ersten Umhüllung (22) umgeben ist, durch die der äußere tubusförmige Körper (2) außerhalb des Körperteils steril gehalten ist.

7. Punktionssystem nach einem der vorhergehenden Ansprüche, **dadurch gekennzeichnet, dass** das Punktionssystem (1), insbesondere dessen Punktionsnadel (5) und/oder dessen innerer tubusförmiger Körpers (4), am patientenfernen Ende (11) ein Aspirationshilfsmittel (7) oder einen Aspirationsanschluss (51) zum Anschluss eines Aspirationshilfsmittels (7) aufweist.

8. Punktionssystem nach einem der vorhergehenden Ansprüche, **dadurch gekennzeichnet, dass** der innere tubusförmige Körper (4) aus Kunststoffmaterial besteht.

9. Punktionssystem nach einem der vorhergehenden Ansprüche, **dadurch gekennzeichnet, dass** der innere tubusförmige Körper (4) länger ist als der äußere tubusförmige Körper (2) und/oder die Punktionsnadel (5) länger ist als der innere tubusförmige Körper (4).

## Claims

1. Puncture system (1) with a three-layer structure, consisting of an outer tubular body (2), which is adapted to dwell in a body part of a living being, an inner tubular body (4) and a puncture needle (5), wherein the inner tubular body (4) is guided through a working lumen (3) of the outer tubular body (2) and is longitudinally displaceable relative to the outer tubular body (2), and the puncture needle (5) is guided through a puncture lumen of the inner tubular body (4) and the inner tubular body (4) is longitudinally displaceable relative to the puncture needle (5), wherein after puncturing of a body part by means of a puncture section (50) of the puncture needle (5) projecting from an end (10) of the puncture system (1) close to the patient, the inner tubular body (4) is at least partially out of the outer tubular body (2) and, at the same time, the puncture section (50) of the puncture needle (5) projecting out of the end (10) of the puncture system (1) close to the patient is designed to be at least partially accommodated in the puncture lumen of the inner tubular body (4), **characterized in that** the outer diameter of the inner tubular body (4) is at most twice as large as the outer diameter of the puncture needle (5) and **in that** the outer diameter of the outer tubular body (2) is at least twice as large as the outer diameter of the inner tubular body (4).

2. Puncture system according to the preceding claim, **characterized in that** a longitudinal displacement of the outer tubular body (2) relative to the inner tubular body (4) and a longitudinal displacement of the inner tubular body (4) relative to the puncture needle (5) are each controllable independently of each other via actuating means (40, 41, 51, 7) from an end (11) of the puncture system (1) remote from the patient.

3. Puncture system according to any one of the preceding claims, **characterized in that** for introducing the outer tubular body (2) into the body part, the puncture system (1) has at the end (10) close to the patient a ramp-like rising dilatation body (21) through which the inner tubular body (4) is guided.

4. Puncture system according to the preceding claim, **characterized in that** the dilatation body (21) is longitudinally displaceable with respect to the inner tubular body (4).

5. Puncture system according to any one of claims 3 to 4, **characterized in that** the dilating body (21) is attached to or is part of the outer tubular body (2).

6. Puncture system according to any one of the preceding claims, **characterized in that** the outer tubular body (2) is surrounded, at least outside the body part, by a first envelope (22) by which the outer tubular body (2) is kept sterile outside the body part.

7. Puncture system according to one of the preceding claims, **characterized in that** the puncture system (1), in particular its puncture needle (5) and/or its inner tubular body (4), has at the end (11) remote from the patient an aspiration aid (7) or an aspiration connection (51) for connecting an aspiration aid (7).

8. Puncture system according to any one of the preceding claims, **characterized in that** the inner tubular body (4) is made of plastic material.

9. Puncture system according to any one of the preceding claims, **characterized in that** the inner tubular body (4) is longer than the outer tubular body (2) and/or the puncture needle (5) is longer than the inner tubular body (4).

## Revendications

1. Système de ponction (1) présentant une structure à trois couches, constitué d'un corps tubulaire extérieur (2) conçu pour rester dans une partie du corps d'un être vivant, d'un corps tubulaire intérieur (4) et d'une aiguille de ponction (5), le corps tubulaire intérieur (4) étant mené à travers une lumière de travail (3) du corps tubulaire extérieur (2) et pouvant se déplacer longitudinalement par rapport au corps tubulaire extérieur (2), et l'aiguille de ponction (5) étant menée à travers une lumière de ponction du corps tubulaire intérieur (4), et le corps tubulaire intérieur (4) pouvant se déplacer longitudinalement par rapport à l'aiguille de ponction (5),
dans lequel, après une ponction d'une partie du corps effectuée au moyen d'une portion de ponction (50) de l'aiguille de ponction (5) dépassant d'une extrémité (10) du système de ponction (1) proche du patient, le corps tubulaire intérieur (4) est conçu de manière à pouvoir sortir au moins partiellement hors du corps tubulaire extérieur (2) par l'extrémité (10) du système de ponction (1) proche du patient et à recevoir au moins partiellement, dans la lumière de ponction du corps tubulaire intérieur (4), la portion de ponction (50) de l'aiguille de ponction (5) dépassant de l'extrémité (10) du système de ponction (1) proche du patient,
**caractérisé en ce que** le diamètre extérieur du corps tubulaire intérieur (4) est au maximum deux fois plus grand que le diamètre extérieur de l'aiguille de ponction (5),
et **en ce que** le diamètre extérieur du corps tubulaire extérieur (2) est au minimum deux fois plus grand que le diamètre extérieur du corps tubulaire intérieur (4).

2. Système de ponction selon la revendication précédente,
**caractérisé en ce qu'**un déplacement longitudinal du corps tubulaire extérieur (2) par rapport au corps tubulaire intérieur (4) et un déplacement longitudinal du corps tubulaire intérieur (4) par rapport à l'aiguille de ponction (5) peuvent être commandés chacun indépendamment l'un de l'autre par des moyens d'actionnement (40, 41, 51, 7) depuis une extrémité (11) du système de ponction (1) éloignée du patient.

3. Système de ponction selon l'une des revendications précédentes,
**caractérisé en ce que**, pour introduire le corps tubulaire extérieur (2) dans la partie du corps, le système de ponction (1) présente à l'extrémité (10) proche du patient un corps de dilatation (21) montant en forme de rampe qui est traversé par le corps tubulaire intérieur (4).

4. Système de ponction selon la revendication précédente,
**caractérisé en ce que** le corps de dilatation (21) est mobile longitudinalement par rapport au corps tubulaire intérieur (4).

5. Système de ponction selon l'une des revendications 3 à 4,
**caractérisé en ce que** le corps de dilatation (21) est fixé au corps tubulaire extérieur (2) ou fait partie du corps tubulaire extérieur (2).

6. Système de ponction selon l'une des revendications précédentes,
**caractérisé en ce que** le corps tubulaire extérieur (2) est entouré, au moins à l'extérieur de la partie du corps, par une première enveloppe (22) grâce à laquelle le corps tubulaire extérieur (2) est maintenu stérile à l'extérieur de la partie du corps.

7. Système de ponction selon l'une des revendications précédentes,
**caractérisé en ce que** le système de ponction (1), en particulier son aiguille de ponction (5) et/ou son corps tubulaire intérieur (4), présente à l'extrémité (11) éloignée du patient un moyen auxiliaire d'aspiration (7) ou un raccord d'aspiration (51) pour raccorder un moyen auxiliaire d'aspiration (7).

8. Système de ponction selon l'une des revendications précédentes,
**caractérisé en ce que** le corps tubulaire intérieur (4) est constitué en matière plastique.

9. Système de ponction selon l'une des revendications précédentes, **caractérisé en ce que** le corps tubulaire intérieur (4) est plus long que le corps tubulaire extérieur (2), et/ou l'aiguille de ponction (5) est plus longue que le corps tubulaire intérieur (4).
